# EUROPEAN PATENT SPECIFICATION

(11) **EP 0 465 091 B1**
(45) Date of publication and mention of the grant of the patent: **17.01.1996**
(21) Application number: 91305729.5
(22) Date of filing: 25.06.1991
(51) Int. Cl.: A61K 31/28

(54) **Use of 3-oxygermylpropionic acid to treat cold syndrome**
Verwendung von 3-Oxygermylpropionsäure zur Behandlung des Erkältungssyndroms
Utilisation de l'acide 3-oxygermylpropionique pour le traitement du syndrome du rhume

(30) Priority: 04.07.1990 JP 176679/90
(43) Date of publication of application: 08.01.1992
(73) Proprietor: SANWA KAGAKU KENKYUSHO CO., LTD., Higashi-ku Nagoya-shi Aichi-ken (JP)
(72) Inventor: Sawai, Kiichi, c/o Sanwa Kagaku Kenk. Co., Ltd., Nagoya-shi, Aichi-ken (JP); Kurono, Masayasu, c/o Sanwa Kagaku Kenk. Co., Ltd., Nagoya-shi, Aichi-ken (JP); Mitani, Takahiko, c/o Sanwa Kagaku Kenk. Co., Ltd., Nagoya-shi, Aichi-ken (JP); Ninomiya, Naohisa, c/o Sanwa Kagaku Kenk. Co., Ltd, Nagoya-shi, Aichi-ken (JP)
(74) Representative: Diamond, Bryan Clive

(56) References cited:
- EP-A- 0 016 444
- EP-A- 0 186 505
- GB-A- 2 190 835
- INT. J. IMMUNOTHERAPY, vol. 1, no. 4, 1985, pages 215-218, Bioscience Ediprint Inc.; Y. SHOJI et al.: "Immunotherapy by combination of immunomodulators and indomethacin"
- JOURNAL OF BIOLOGICAL RESPONSE MODIFIERS, vol. 8, no. 2, 1989, pages 180-189, Raven Press, Ltd, New York, US; H. ASO et al.: "Antiviral activity of carboxyethylgermanium sesquioxide (Ge-132) in mice infected with influenza virus"
- PATENT ABSTRACTS OF JAPAN, vol. 10, no. 43 (C-329)[2100], 20th Februay 1986; & JP-A-60 190 714
- MEDICAL HYPOTHESES, vol. 26, no. 3, June 1988, pages 207-215, Longman Group UK Ltd, GB; S. GOODMAN: "Therapeutic effects of organic germanium"

## Description

The present invention relates to the use of a composition comprising 3-oxygermylpropionic acid (3-OGP for short) of the formula:

[(O_{1/2})₃GeCH₂CH₂COOH]ₙ

wherein n is an integer of 1 or more, and a cellulosic carrier, used to treat cold syndrome.

The 3-OGP in the form of a white acicular crystal has physicochemical properties expressed in terms of a specific gravity or density of 2.23, a solubility in water of 1.57 at 20°C and a melting or decomposition point of about 230.

The pharmacological activity of this compound, because it has polymerizability and a variety of uses, has recently attracted attention, e.g. our EP-A-444931 describes use for treating diabetes-dependant diseases. It has been suggested that it is possibly usable for treating viral colds. However, a serious problem with 3-OGP is that it is so unstable in the presence of water that its pharmacological activity decreases drastically (see JP-A-57-53800 and US-A-4309412).

For treating cold syndrome, on the other hand, antipyretics, antiphlogistics and analgesics based on weak acid compounds, such as aspirin, indomethacin (U.S.P. 3161654) and ibuprofen (U.S.P 3228831) and antihistamines have generally been used. However, use of these medicaments in combination with organic germanium compounds cannot be expected to produce sufficient pharmacological effects, since they are likely to undergo changes in composition under the influence of moisture.

We have studied not only compositions for enabling 3-OGP to maintain its own pharmacological activity stably but how it acts in vivo as well. We have thus already found that a variety of substances can be effectively used as stabilizers for 3-OGP (see JP-A-61-65819) and that saccharides can enhance its activity (see JP-A-60-190714). We have also discovered that 3-OGP has immuno-modulatory actions (see JP-A-61-151123).

UK-A-2190835 discloses an organogermanium compound for administration to patients suffering from the consequences of cold syndrome. In addition, EP-A-0016444 discloses a carboxyethyl germanium oxide as an agent for inducing interferon. Also, EP-A-0186505 discloses pharmaceutical compositions containing an organogermanium compound used for influencing the human immune system.

Further, Hisashi Aso et al (Journal of Biological Response Modifiers 8(2), 1989 pp. 180-189) describe the antiviral activity of carboxyethylgermanium sesquioxide in mice infected with influenza virus. A drug composition comprising, as active ingredient, an organic germanium compound is disclosed, in Patent Abstracts of Japan, 10(43), (C-329) [2100], 20th February 1986 and in JP-A-60190714, which is useful as a remedy for viral disease or inflammation. Also, the therapeutic effects of organic germanium, emphasising germanium's anti-viral and immunological properties, is taught in Medical Hypotheses, 26(3), June 1988, pp. 207-215.

When receiving antipyretics, antiphlogistics and/or analgesics, patients with cold syndrome are likely to suffer gastrointestinal or other disorders due to their reduced. strength. On rare occasions, they are reported to have suffered anaphylaxis incidental to immunodeficiency or such disorders as acute hepatic insufficiency.

Cold syndrome per se is caused not only by viral infection but also by weather changes and physical conditions, giving rise to unusual immunoconditions, and its whole aspect has yet to be elucidated.

No special therapy is still established for cold syndrome by reason of no dire need of treatment. However, early treatment is required for colds, since they are said to "develop into all kinds of illness" and are thus likely to accompany the diversity of secondary diseases.

We have now found that 3-OGP, which is of great safety and has such physicochemical properties as already mentioned, provides a composition greatly stable during storage and showing highly stabilized activity in vivo when it is used in combination with a cellulosic activating carrier. In a preferable composition used, 0.005 to 50% by weight of hydroxypropylcellulose as an activating carrier are used in combination with 0.005 to 20% by weight of 3-OGP. We have also found that medicaments, which are obtained by mixing such a composition with generally available antipyretics, antiphlogistics and/or analgesics for colds, are stable in composition and can maintain their pharmacological activities well after administration to human patients, while suppressing the toxicities of such drugs.

According to the invention we provide the use of a combination of 3-oxygermylpropionic acid and hydroxypropylcellulose for the manufacture of a medicament to treat cold syndrome, wherein the hydroxypropylcellulose is used as an activating carrier in an amount of 0.005 to 50% by weight per 0.005 to 20% by weight of the 3-oxygermylpropionic acid.

In the accompanying drawings:
Figure 1 is a graph showing the effect of an ethanol-treated composition of hydroxypropylcellulose and 3-OGP upon the treatment of the adjuvant-induced arthritis and secondary inflammation of rats, and
Figure 2 is a graph showing the effect of a composition consisting only of hydroxypropylcellulose and 3-OGP upon the treatment of the adjuvant-induced arthritis and secondary inflammation of rats.

The present pharmaceutical composition for treating cold syndrome may be used in usual forms. In some cases, however, it should be an enteric coated pill in view of the antipyretics, antiphlogistics and/or analgesics incorporated.

The antipyretics, antiphlogistics and/or analgesics used include frequently used drugs, e.g. aspirin, ibuprofen and acetaminophen. In addition, if such drugs as reported to have side effects, e.g. indomethacin, flurbiprofen (US-A-3755427) and mefenamic acid are used in combination with 3-OGP, then it is possible to suppress their toxicities for safe administration.

Although the dose varies dependent upon the type of compound, the type of composition, the age of patient and other factors, the composition of this invention, when used in combination with antipyretics, antiphlogistics and analgesics, may generally be administered to human patients at a dose between 10 mg/kg and 1500 mg/kg. A preferable oral daily dose to adult patients (weight 50 kg) is about 150 mg.

### EXAMPLES

The present invention will now be illustrated by the following examples.

### Preparation Example 1 (Preparation of Composition for Activating 3-OGP)

Hydroxypropylcellulose as carrier and 3-OGP were mixed together at a weight ratio 1:2. The mixture was kneaded, with ethanol serving as a wetting agent, and then dried at a temperature lower than 50°C to obtain a powdery or granular primary composition.

### Preparation Example 2

The primary composition was blended according to the following formulation, and tabletted in conventional manner.

| Ingredients | Amount (mg) |
|---|---|
| Primary composition | 100 |
| Lactose | suitable |
| Methylcellulose | 20 |
| Hydroxyproylcellulose | 8 |
| Sucrose fatty acid ester | 2 |
| Magnesium stearate | 2 |
| Sodium lauryl sulfate | 1 |
| | 200 mg per tablet |

### Preparation Example 3

| Ingredients | Amount (mg) |
|---|---|
| Primary composition | 100 |
| Indomethacin | 20 |
| Lactose | suitable |
| Methylcellulose | 20 |
| Hydroxyproylcellulose | 8 |
| Sucrose fatty acid ester | 2 |
| Magnesium stearate | 2 |
| Sodium lauryl sulfate | 1 |
| | 200 mg per tablet |

### Pharmacological Tests

### I. Effect of incorporated hydroxypropylcellulose

Investigations were made of the blending ratio and condition of 3-OGP and hydroxypropylcellulose and the action of the resulting blend upon suppressing immunoinflammation.

### A) Compositions

(a) Simple composition obtained by mere blending of 3-OGP with hydroxypropylcellulose.
(b) Composition prepared by kneading 3-OGP with hydroxypropylcellulose under conditions wetted by a solvent (ethanol) and drying and pulverizing the product at a temperature no higher than 50 .

The above-mentioned compositions contained hydroxypropylcellulose and 3-OGP at weight ratios 1:2 (77% composition), 1:1 (50% composition), 2:2 (133% composition) and 4:1 (20% composition).

An adjuvant (a suspension of 0.6 mg of Myco. butyricum in 0.05 ml of liquid paraffin) was subcutaneously administered at a dose of 0.05 ml to the plantae of the hind legs of groups of a Sprague-Dawley line of (masculine) rats (weights: 170-210 g), five for each group. At 1, 3, 5, 7, 14, 21 and 28 days from adjuvant dosing, the volumes of the plantae of the forelegs were measured to find the ratio of edemas. The animals received various compositions at a daily dose of 1 mg/kg, calculated as 3-OGP, over a period of 28 days.

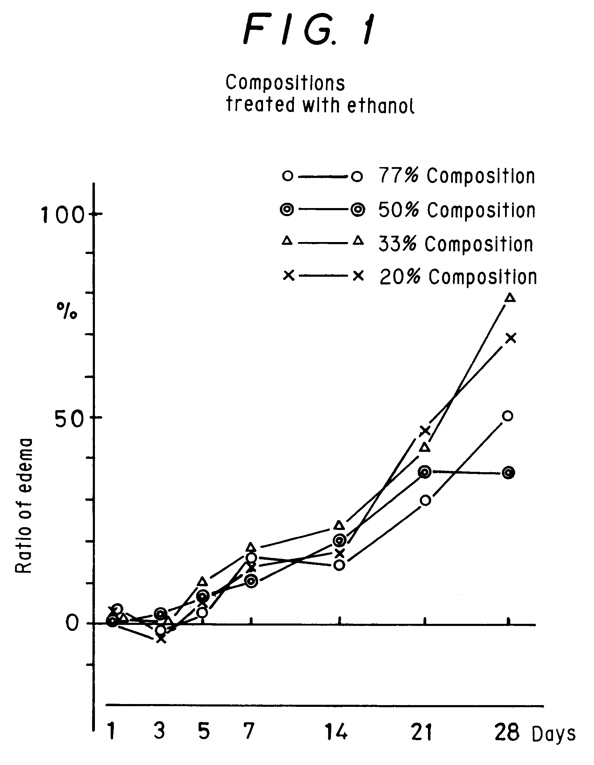

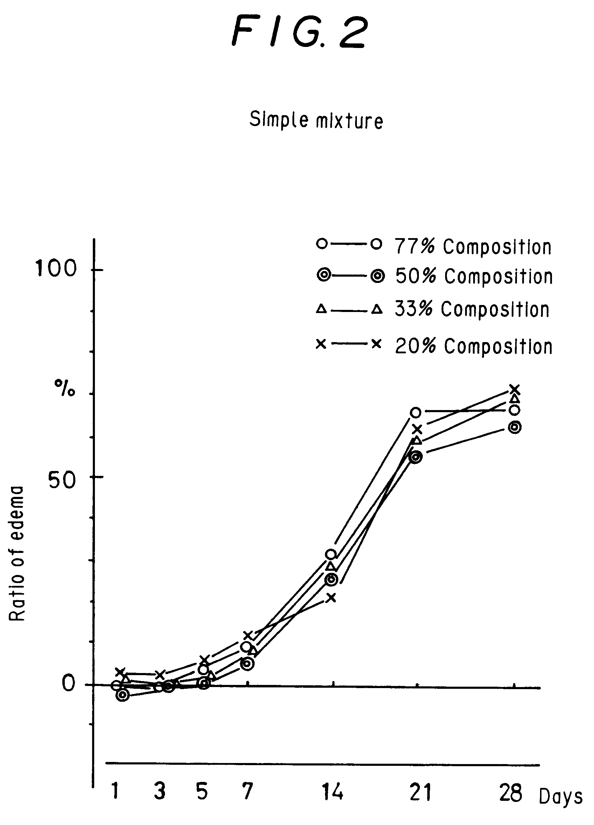

As can be understood from the results shown in Figs. 1 and 2, some compositions (b) containing 50 % or more of 3-OGP have a great effect upon suppressing secondary inflammation during the period of the 14th - 28th days.

### II. Effect on relieving the toxicity of indomethacin

### a) Influence on the amount of urine and excretion of electrolytes

It has been reported that antipyretics, antiphlogistics and analgestics have side effects because they tend to inhibit the synthesis of prostaglandin playing an important role in maintaining bloodstream in the kidney, giving rise to decreases in the amount or urine and the amount of Na⁺ excreted. See "Adv. Inflammation Res.", 6, pp. 149-158, 1984.

Thus, investigation was made of whether or not the 3-OGP compositions are efficacious against the permeation through the kidney of indomechacin reported to be likely to permeate into the tissue of the kidney.

The drugs to be tested were orally administered to groups of a Wister line or (masculine) rats (weights: 170-200g) - six for each group - after they had been fasted. Just after that, the animals received 2.5 ml/100 g of a physiological saline solution, and then placed in urine collectors one by one. Urine was collected every hour over 5 hours to measure the amount of urine and find the amount of electrolytes (Na⁺ and Ka⁺) in urine by the use of a flame photometer (Type 480; made by Corning Co., Ltd.), thereby determining the Na⁺/Ka⁺ ratio.

The drugs used were:
(1) 15 mg/Kg (10 mg/kg calculated as 3-OGP) of the primary composition according to Preparation Example 1;
(2) 100 g/Kg of an organogermanium compound SK 818;
(3) 10 mg/Kg of Indomethacin (IND);
(4) 15 mg/Kg of (10 mg/Kg calculated as 3-OGP) of the primary composition according to Preparation Example 1 and 10 mg/Kg of indomethacin - administer at once;
(5) Administration of a physiological saline solution - control; and
(6) 40 mg/Kg of furosemide (a diuretic)

### Results

As shown in Table 1, the use of the 3-OGP composition in combination with indomethacin serves to inhibit the decreases in the amount of urine and the amount of electrolytes excreted in urine, caused by indomethacin.

**Table 1**

| | Amount of Urine (ml/5hr) | Na⁺/Ka⁺ |
|---|---|---|
| Primary composition (15mg/Kg) | 1.94 0.24 | 2.0 0.3 |
| SK 818 (100mg/Kg) | 1.90 0.36 | 1.5 0.2 |
| IND (10mg/Kg) | 1.14 0.12 | 1.2 0.5 |
| Primary composition (15mg/Kg) + IND (10mg/Kg) | 2.11 0.32 | 1.7 0.3 |
| Control | 1.95 0.36 | 1.7 0.2 |
| Furosemide (Diuretic) | 13.53 0.48 | 4.9 0.2 |

### b) Antipyretic Activity

A 20 % suspension of dry yeast was subcutaneously injected into a Wister line of (masculine) rats (weights: 170-200 g) at a dose of 1 ml/100 g to bring their rectal temperatures up to higher than 39°C(39.13 0.02 ) after the lapse of 18 hours.

The drugs to be tested were orally administrated to these rats, six for each group, to measure their rectal temperatures at 1, 2, 4 and 8 hours from dosing.

The drugs used were:
(1) 75 mg/Kg (50 mg/Kg calculated as 3-OGP) of the primary composition according to Preparation Example;
(2) 1 mg/Kg of indomethacin (IND);
(3) 5 mg/Kg of indomethacin (IND);
(4) 75 mg/Kg (50 mg/Kg calculated as 3-OGP) of the primary composition according to Preparation Example 1 + 1 mg/Kg of indomethacin - administer at once; and
(5) No drug - control.

### Results

As shown in Table 2, the administration of 50 mg/Kg of 3-OGP in combination with 1 mg/Kg of IND shows a remarkable antipyretic effect. In other words, the 3-OGP composition serves to enhance the antipyretic action of indomethacin.

**Table 2**

| Changes in mean temperature | | | | | |
|---|---|---|---|---|---|
| Time, hr. | 0 | 1 | 2 | 4 | 8 |
| Primary composition (75mg/Kg) | 39.13 | 39.01 | 38.92 | 38.81 | 38.65 |
| | 0.02 | 0.03 | 0.10 | 0.24 | 0.17 |
| IND (1mg/Kg) | 39.13 | 38.81 | 38.65 | 38.44 | 38.30 |
| | 0.02 | 0.29 | 0.31 | 0.22 | 0.21 |
| IND (5mg/Kg) | 39.13 | 38.35 | 37.67 | 37.75 | 37.94 |
| | 0.02 | 0.32 | 0.42 | 0.31 | 0.31 |
| Primary Composition (75mg/Kg) + IND (1mg/Kg) | 39.13 | 38.43 | 37.61 | 37.91 | 38.01 |
| | 0.02 | 0.16 | 0.32 | 0.18 | 0.21 |
| Control | 39.13 | 39.11 | 39.00 | 38.90 | 38.82 |
| | 0.02 | 0.03 | 0.01 | 0.02 | 0.02 |

### Questionnairing

Five pre-registered volunteers were allowed to take a commercially available cold remedy (for instance, "Kazemin®" prepared by Sanwa Kagaku Kenkyusyo Co. Ltd. and based on acetaminophen, caffeine and vitamin C) at their disposal, with the tablets according to Preparation Example 2 at a dose of up to 3 tablets a day, when they diagnosed themselves as having a cold.

Questioning was then conducted about to what extent they were affected in comparison with their last conditions (i.e. to what extent throat ache, nasal congestion, coughing, removal of fever and headache were affected). All the subjects responded that their conditions were early affected.

It is generally reported that even those who are in good health condition contract cold about 1 to 5 times a year, and the condition starts with throat inflammation that lasts for 2 or days, followed by naval congestion, fever and bronchopneumonia. For cure, one or two weeks are said to be needed.

Colds can be caused by many factors inclusive of environmental changes, virus and immunoabnormality. However, there is no experimental reproducibility or a difference between individuals regarding what condition they are in. For these reasons and other factors such as placebo effects and use of the commercially available cold remedy, the above-mentioned results may somewhat lack reliability. However, it is believed that the 3-OGP composition is efficacious against cold to some degree.

## Claims

1. Use of a combination of 3-oxygermylpropionic acid and hydroxypropylcellulose for the manufacture of a medicament to treat cold syndrome, wherein the hydroxypropylcellulose is used as an activating carrier in an amount of 0.005 to 50% by weight per 0.005 to 20% by weight of the 3-oxygermylpropionic acid.

2. Use according to Claim 1, wherein said acid is used in a composition in conjunction with an antipyretic, an antiinflammatory and/or an analgesic.

3. Use according to any preceding Claim, wherein the acid is used in an amount of 10 mg/Kg to 1500 mg/Kg daily dose per human patients.

## Patentansprüche

1. Verwendung einer Kombination von 3-Oxygermylpropionsäure und Hydroxypropylzellulose für die Herstellung eines Medikaments zur Behandlung des Erkältungssyndroms, bei der die Hydroxypropylzellulose als Aktivierungsträger in einer Menge von 0,005 bis 50 Gew.% pro 0,005 bis 20 Gew.% von 3-Oxygermylpropionsäure benutzt wird.

2. Verwendung nach Anspruch 1, bei der die genannte Säure in einer Zusammensetzung in Verbindung mit einem fieberhemmenden Mittel, einem entzündungshemmenden Mittel und/oder einem Analgetikum benutzt wird.

3. Verwendung nach einem der vorhergehenden Ansprüche, bei der die Säure in einer Menge von 10 mg/kg bis 1500 mg/kg Tagesdosis für menschliche Patienten benutzt wird.

## Revendications

1. Utilisation d'une combinaison d'acide 3-oxygermylpropionique et d'hydroxypropylcellulose pour la fabrication d'un médicament pour le traitement du syndrome du rhume, dans laquelle l'hydroxypropylcellulose sert de véhicule activateur en une quantité de 0,005 à 50 % en poids pour 0,005 à 20 % en poids de l'acide 3-oxygermylpropionique.

2. Utilisation selon la Revendication 1, dans laquelle l'acide est utilisé dans une composition conjointement à un fébrifuge, un anti-inflammatoire et/ou un analgésique.

3. Utilisation selon l'une ou l'autre des Revendications qui précèdent, dans laquelle l'acide est utilisé en une quantité de 10 mg/kg à 1500 mg/kg en dose journalière par patient humain.
